# EUROPEAN PATENT APPLICATION

(11) **EP 2 606 895 A1**
(43) Date of publication of application: **26.06.2013**
(21) Application number: 10838390.2
(22) Date of filing: 12.10.2010
(51) Int. Cl.: A61K 31/513, A61K 31/522, A61K 9/70, A61P 31/12

(54) **TRANSDERMAL ABSORPTION PATCH OF ANTIVIRAL DRUG AND ITS PREPARATION METHOD**

(71) Applicant: Wuhan University, Hubei 430072 (CN)
(72) Inventor: WANG, Yefu, Wuhan Hubei 430072 (CN); LI, Wei, Wuhan Hubei 430072 (CN); ZHOU, Xiaozhou, Wuhan Hubei 430072 (CN)
(74) Representative: Zeuner, Stefan
(86) International application number: PCT/CN2010/077636
(87) International publication number: WO 2012/048455

(57) **Abstract**

An antiviral transdermal absorbing patch includes a backing layer, a viscous polymer layer, and a protection film layer. The viscous polymer layer includes an antiviral agent, a viscous polymer, and a transdermal enhancer. The transdermal enhancer is laurocapram or a mixture thereof. The antiviral agent is a nucleoside antiviral drug with a daily delivery rate of less than 100 mg/day. A method for producing the patch is also provided. The antiviral transdermal absorbing patch effectively promotes the penetration of a nucleoside antiviral agent into skin and then the agents is assimilated via capillary vessels and enters the blood circulation, thereby inhibiting the replication of target viruses and reducing viral DNA level in the serum, and administrating sustainably and stably. In addition, the transdermal administration avoids the enzymolysis of gastrointestinal tract and the first pass effect of the liver and reduces toxicity and side effect of drugs. The raw materials involved in the invention are simple and available from the marke , with a low cost, which enable the patch to have the great practibility.

## Description

### FIELD OF THE INVENTION

The invention relates to a patch via transdermal administration,, and more particularly to an antiviral transdermal absorbing patch. The invention also relates to a method for producing the antiviral transdermal absorbing patch.

### BACKGROUND OF THE INVENTION

Currently, an antiviral drug that is used the most widely and has the most variety is nucleoside antiviral drug, which includes entecavir, adefovir, lamivudine, acyclovir, ribavirin, zidovudine and so on. The nucleoside antiviral drug is usually used for treatment of viral diseases such as hepatitis B, AIDS, influenza virus, and herpes,

Dosage forms of the nucleoside antiviral drug generally include oral formulations or injections. However, The oral administration mode or the injection administration mode often causes a problem that blood concentration of active ingredients is markedly fluctuated with time. That is to say, too low concentration of active ingredients in blood cannot exhibit desired effects, too high concentration of active ingredients in blood, however, may be poisonous to human body due to overdosage. Moreover, the oral administration mode or the injection administration mode still exist such problems as enzymolysis of administered drugs in the gastrointestinal tract and the first pass effect of the liver, thereby reducing the efficacy.

### SUMMARY OF THE INVENTION

Accordingly, an object of the present invention to provide an antiviral transdermal absorbing patch that could deliver sustainably and stably, and also could eliminate enzymolysis of administered drugs in the gastrointestinal tract and the first pass effect of the liver, and reduce toxicity and side effects.

To achieve the above-mentioned object, the present invention provides an antiviral transdermal absorbing patch comprising a backing layer, a viscous polymer layer, and a protection film layer, wherein the viscous polymer layer comprises an antiviral agent, a viscous polymer, and a transdermal enhancer.

As an embodiment of the present invention, the antiviral agent is a nucleoside antiviral drug with a daily administration rate of less than 100 mg/day.

As another embodiment of the present invention, the nucleoside antiviral drug with a daily administration rate of less than 100 mg/day is selected from the group consisting of entecavir, adefovir dipivoxil, lamivudine, stavudine, or a mixture thereof.

As still another embodiment of the present invention, the content of the antiviral agent per square centimeter of the antiviral transdermal absorbing patch is between 0.1 and 50 mg, preferably between 1.5 and 40 mg, more preferably between 5 and 30 mg, particularly between 10 and 25 mg, and more particularly between 15 and 20 mg.

As still another embodiment of the present invention, the antiviral agent of the antiviral transdermal absorbing patch accounts for between 0.1 and 15.0 wt.% of the viscous polymer layer, preferably between 0.5 and 13.0 wt.%, particularly between 1.0 and 9.0 wt.%, and more particularly between 5.0 and 7.0 wt.%.

As still another embodiment of the present invention, the viscous polymer is selected from the group consisting of a polyacrylate pressure sensitive adhesive, polyisobutylene, polyisoprene, and a silicone copolymer.

As still another embodiment of the present invention, the polyacrylate pressure sensitive adhesive is selected from the group consisting of polyacrylic resin II, polyacrylic resin III, polyacrylic resin IV, ammonio methacrylate copolymer I, ammonio methacrylate copolymer II, and acrylic resin-EUDRAGIT E100; the acrylic resin-EUDRAGIT E100 is a copolymer of butyl methacrylate-dimethylamino ethyl methacrylate-methyl methacrylate (1:2:1).

As still another embodiment of the present invention, the viscous polymer accounts for between 50.0 and 96.0 wt.% of the viscous polymer layer, preferably between 65.0 and 90.0 wt.%, particularly between 75.0 and 85 wt.%, and more particularly between 78.0 and 82.0 wt.%.

As still another embodiment of the present invention, the transdermal enhancer is selected from the group consisting of laurocapram, essential oils, dimethyl sulfoxide, thymol, eucalyptus oil, and a traditional chinese medicine comprising terpenes or phenols or a mixture thereof.

Preferably, the transdermal enhancer is laurocapram.

Also preferably, the transdermal enhancer is a mixture of laurocapram and essential oil with a weight ratio of 1: 0.5-2.

Also preferably, the transdermal enhancer is a mixture of laurocapram and dimethyl sulfoxide with a weight ratio of 1: 0.5-2.

As still another embodiment of the present invention, the transdermal enhancer accounts for between 2.0 and 12.0 wt.% of the viscous polymer layer, preferably between 4.0 and 11.0 wt.%, particularly between 6.0 and 10 wt.%, and more particularly between 8.0 and 9.0 wt.%.

As still another embodiment of the present invention, the viscous polymer layer further comprises allowable organic solvent, for example, propylene glycol or ethyl lactate.

As still another embodiment of the present invention, an adhesive layer is disposed between the viscous polymer layer and the protection film layer.

As still another embodiment of the present invention, a raw material of the adhesive layer is selected from a polyacrylate pressure sensitive adhesive, polyisobutylene, or a silicone copolymer.

As still another embodiment of the present invention, the backing layer of the antiviral transdermal absorbing patch is selected from the group consisting of high-density polyethylene, low density polyethylene, polypropylene, polyvinyl chloride, an ethylene-vinyl acetate copolymer, polyester, polyvinylpyrrolidone, polyvinyl alcohol, poly urethane, aluminum foil, or a mixture thereof.

As still another embodiment of the present invention, the backing layer is a composite film formed by reaction of an aluminum foil with high-density polyethylene, low density polyethylene, polypropylene, polyvinyl chloride, ethylene-vinyl acetate copolymer, polyester, polyvinylpyrrolidone, polyvinyl alcohol, poly urethane, or a mixture thereof.

As still another embodiment of the present invention, the backing layer is a three-layered composite film with an aluminum foil in the middle and low density polyethylene, poly urethane, or a mixture thereof at both sides.

The present invention also provides a method for producing the antiviral transdermal absorbing patch comprising the steps of a) uniformly mixing the antiviral agent, the viscous polymer, the transdermal enhancer, and a solvent to yield a mixture; b) coating the mixture to the backing layer to yield the viscous polymer layer; c) drying the viscous polymer layer and then cooling the viscous polymer layer; and d) after cooling, coating the protection film layer to the other surface of the viscous polymer layer to yield the antiviral transdermal absorbing patch.

The invention also provides application of the antiviral transdermal absorbing patch in the manufacture of antiviral drug.

Advantages of the invention are summarized below.

In the invention, the viscous polymer layer let the viscous polymer function as a delayed-release framework material, and after a specific amount of antiviral agent is dissolved in the transdermal enhancer which is laurocapram or a mixture thereof, uniformly distribute the transdermal enhancer with the antiviral agent dissolved therein in the framework in the form of liquid micro-rooms through solvent evaporation. Then control the concentration of drugs, the density of the liquid micro-rooms, and the proportion of the transdermal enhancer to regulate the release rate of the antiviral agent of the patch.

The antiviral transdermal absorbing patch of the invention which is made from laurocapram or a mixture thereof functioning as the transdermal enhancer could effectively promotes the penetration of the nucleoside antiviral agent into skin, and then the agents is assimilated via capillary vessels and enters the blood circulation, thereby inhibiting the replication of target viruses and reducing viral DNA level in the serum. In addition, the transdermal administration avoids the enzymolysis of gastrointestinal tract and the first pass effect of the liver and reduces toxicity and side effect of drugs. In the invention, as laurocapram or a mixture thereof functions as the transdermal enhancer, which could effectively promotes the penetration of the nucleoside antiviral agent into skin, thus the resulting antiviral transdermal absorbing patch of the invention can releases active ingredients sustainably and stably for 3-5 consecutive days. The raw materials involved in the invention are simple and available from market, with a low cost, which enable the patch to have the great practibility.

Pharmacology and toxicology research on the antiviral transdermal absorbing patch of the invention have shown that allergy test and skin irritation test of the patch is negative. That is to say, the antiviral transdermal absorbing patch has no irritation on skin, and no hemolysis and allergy occur. Thus, the patch is safe.

Moreover, Clinical studies have shown that the antiviral transdermal absorbing patch of the invention accelerates the penetration of nucleoside antiviral agent into skin, and the efficacy can be maintained for 3-5 consecutive days, thus the time of administration is reduced. Therefore, the antiviral transdermal absorbing patch of the invention is a safe, long-term effective and convenient transdermal absorbing formulation for treatment of viral diseases.

### DETAILED DESCRIPTION OF ILLUSTRATED EMBODIMENTS

For further illustrating the invention, experiments detailing an antiviral transdermal absorbing patch and a method for producing the same are described below. It should be noted that the following examples are intended to describe and not to limit the invention.

The following is the first example.

To a jar, 185 g of polyacrylate pressure sensitive adhesive (EUDRAGIT E100, from Germany, pre-dissolved with ethyl acetate, in which solid EUDRAGIT E100 accounts for 40 wt.%) as a viscous polymer, 0.5 g of entecavir as an antiviral agent, 9 g of laurocapram as a transdermal enhancer, a mixture of 6 g of eucalyptus oil and 8.9 g of propylene glycol, and 100 g of ethyl acetate as a solvent were added. The jar was sealed and oscillated on the oscillator for 15 hrs, and then stood until bubbles disappeared. The resulting mixture was coated to a backing layer comprising polythene-aluminum-polyethylene to yield a viscous polymer layer which was further dried at 40°C for 4 min, at 60°C for 2 min, at 90°C for 2 min, and then cooled. A non-adhesive paper was coated on the other surface of the viscous polymer layer. The product was cut into patches with an area of 2 cm² to yield an antiviral transdermal absorbing patch I with 2.5 mg of entecavir per square centimeter. The antiviral transdermal absorbing patch I comprised 75.2 wt.% polyacrylate pressure sensitive adhesive, 0.5 wt.% entecavir, 9.1 wt.% laurocapram, 6.1 wt.% eucalyptus oil, and 9 wt.% propylene glycol. The permeation rate of the patch measured using upgraded Franz diffusion cell was 15 µg/cm²/h, and the release time of the active ingredients exceeded 72 hrs. Thus, the patch was suggested for use of up to 3 days.

The following is the second example.

To a jar, 225 g of polyacrylate pressure sensitive adhesive (EUDRAGIT E100, from Germany, pre-dissolved with ethyl acetate, in which solid EUDRAGIT E100 accounts for 40 wt.%) as a viscous polymer, 10 g of adefovir dipivoxil as an antiviral agent, a mixture of 9 g of laurocapram and 3 g of thymol as a transdermal enhancer, and 150 g of dichloromethane as a solvent were added. The jar was sealed and oscillated on the oscillator for 15 hrs, and then stood until bubbles disappeared. The resulting mixture was coated to a backing layer comprising low-density polyethylene to yield a viscous polymer layer which was further dried at 40°C for 4 min, at 60°C for 2 min, at 90°C for 2 min, and then cooled. A non-adhesive paper was coated on the other surface of the viscous polymer layer. The product was cut into patches with an area of 20 cm² (4 cm × 5 cm) to yield an antiviral transdermal absorbing patch II with 35 mg of adefovir dipivoxil per square centimeter. The antiviral transdermal absorbing patch II comprised 80.4 wt.% polyacrylate pressure sensitive adhesive, 9 wt.% adefovir dipivoxil, 8 wt.% laurocapram, and 2.6 wt.% thymol. The permeation rate of the patch measured using upgraded Franz diffusion cell was 20 µg/cm²/h, and the release time of the active ingredients exceeded 72 hrs. Thus, the patch was suggested for use of up to 3 days.

The following is the second example.

To a jar, 128 g of polyisobutylene as a viscous polymer, 14 g of lamivudine as an antiviral agent, a mixture of 6 g of laurocapram and 10 g of essential oil as a transdermal enhancer, 42 g of ethyl lactate, and 134 g of ethyl acetate as a solvent were added. The jar was sealed and oscillated on the oscillator for 15 hrs, and then stood until bubbles disappeared. The resulting mixture was coated to a backing layer comprising poly urethane to yield a viscous polymer layer which was further dried at 40°C for 4 min, at 60°C for 2 min, at 90°C for 2 min, and then cooled. A non-adhesive paper was coated on the other surface of the viscous polymer layer. The product was cut into patches with an area of 200 cm² to yield an antiviral transdermal absorbing patch III with 15 mg of lamivudine per square centimeter. The antiviral transdermal absorbing patch III comprised 64 wt.% polyisobutylene, 7 wt.% lamivudine, 3 wt.% laurocapram, 5 wt.% essential oil, and 21 wt.% ethyl lactate. The permeation rate of the patch measured using upgraded Franz diffusion cell was 55 µg/cm²/h, and the release time of the active ingredients exceeded 96 hrs. Thus, the patch was suggested for use of up to 4 days.

The following is the fourth example.

To a jar, 87.5 g of polyisoprene as a viscous polymer, 15 g of stavudine as an antiviral agent, a mixture of 6 g of laurocapram and 6 g of dimethyl sulfoxide as a transdermal enhancer, and 147 g of ethyl acetate as a solvent were added. The jar was sealed and oscillated on the oscillator for 15 hrs, and then stood until bubbles disappeared. The resulting mixture was coated to a backing layer comprising poly urethane to yield a viscous polymer layer which was further dried at 40°C for 4 min, at 60°C for 2 min, at 90°C for 2 min, and then cooled. A non-adhesive paper was coated on the other surface of the viscous polymer layer. The product was cut into patches with an area of 100 cm² to yield an antiviral transdermal absorbing patch IV with 10 mg of stavudine per square centimeter. The antiviral transdermal absorbing patch IV comprised 76.5 wt.% polyisoprene, 13.1 wt.% stavudine, 5.2 wt.% laurocapram, and 5.2 wt.% dimethyl sulfoxide. The permeation rate of the patch measured using upgraded Franz diffusion cell was 34 µg/cm²/h, and the release time of the active ingredients exceeded 72 hrs. Thus, the patch was suggested for use of up to 3 days.

The following is the fifth example.

To a jar, 88 g of silicone copolymer as a viscous polymer, 6 g of adefovir dipivoxil as an antiviral agent, a mixture of 7 g of laurocapram and 4.4 g of menthol as a transdermal enhancer, and 147 g of ethyl acetate were added. The jar was sealed and oscillated on the oscillator for 15 hrs, and then stood until bubbles disappeared. The resulting mixture was coated to a backing layer comprising poly urethane to yield a viscous polymer layer which was further dried at 40°C for 4 min, at 60°C for 2 min, at 90°C for 2 min, and then cooled. A non-adhesive paper was coated on the other surface of the viscous polymer layer. The product was cut into patches with an area of 30 cm² to yield an antiviral transdermal absorbing patch V with 40 mg of adefovir dipivoxil per square centimeter. The antiviral transdermal absorbing patch V comprised 83.5 wt.% silicone copolymer, 5.7 wt.% adefovir dipivoxil, 6.6 wt.% laurocapram, and 4.2 wt.% menthol. The permeation rate of the patch measured using upgraded Franz diffusion cell was 18 µg/cm²/h, and the release time of the active ingredients exceeded 72 hrs. Thus, the patch was suggested for use of up to 3 days.

The following is the sixth example.

To a jar, 225 g of polyacrylate pressure sensitive adhesive (EUDRAGIT E100, from Germany, pre-dissolved with ethyl acetate, in which solid EUDRAGIT E100 accounts for 40 wt.%) as a viscous polymer, 0.5 g of entecavir as an antiviral agent, 3.77 g of laurocapram as a transdermal enhancer, and 147 g of ethyl acetate as a solvent were added. The jar was sealed and oscillated on the oscillator for 15 hrs, and then stood until bubbles disappeared. The resulting mixture was coated to a backing layer comprising poly urethane to yield a viscous polymer layer which was further dried at 40°C for 4 min, at 60°C for 2 min, at 90°C for 2 min, and then cooled. A layer of polyacrylate was coated on the other surface of the viscous polymer layer, dried and cooled following the process mentioned above, and then a non-adhesive paper was coated. The product was cut into patches with an area of 4 cm² (2 cm × 2 cm) to yield an antiviral transdermal absorbing patch VI with 3.25 mg of entecavir per square centimeter. The antiviral transdermal absorbing patch VI comprised 95.5 wt.% polyacrylate pressure sensitive adhesive, 0.5 wt.% entecavir, and 4 wt.% laurocapram. The permeation rate of the patch measured using upgraded Franz diffusion cell was 10 µg/cm²/h, and the release time of the active ingredients exceeded 48 hrs. Thus, the patch was suggested for use of up to 2 days.

The following is the seventh example.

To a jar, 225 g of polyacrylate pressure sensitive adhesive (EUDRAGIT E100, from Germany, pre-dissolved with ethyl acetate, in which solid EUDRAGIT E100 accounts for 40 wt.%) as a viscous polymer, 0.2 g of entecavir as an antiviral agent, 9.8 g of laurocapram as a transdermal enhancer, 6.38 g of propylene glycol, and 160 g of ethyl acetate as a solvent were added. The jar was sealed and oscillated on the oscillator for 15 hrs, and then stood until bubbles disappeared. The resulting mixture was coated to a backing layer comprising polythene-aluminum-polyethylene to yield a viscous polymer layer which was further dried at 40°C for 4 min, at 60°C for 2 min, at 90°C for 2 min, and then cooled. A layer of silicone copolymer was coated on the other surface of the viscous polymer layer, dried and cooled following the process mentioned above, and then a non-adhesive paper was coated. The product was cut into patches with an area of 4 cm² (2 cm × 2 cm) to yield an antiviral transdermal absorbing patch VII with 2.2 mg of entecavir per square centimeter. The antiviral transdermal absorbing patch VII comprised 84.6 wt.% polyacrylate pressure sensitive adhesive, 0.19 wt.% entecavir, 9.21 wt.% laurocapram, and 6 wt.% propylene glycol. The permeation rate of the patch measured using upgraded Franz diffusion cell was 12 µg/cm²/h, and the release time of the active ingredients exceeded 72 hrs. Thus, the patch was suggested for use of up to days.

The following is the eighth example.

To a jar, 170 g of polyacrylate pressure sensitive adhesive (EUDRAGIT E100, from Germany, pre-dissolved with ethyl acetate, in which solid EUDRAGIT E100 accounts for 40 wt.%) as a viscous polymer, 0.8 g of entecavir as an antiviral agent, a mixture of 4 g of laurocapram and 5.4 g of eucalyptus oil as a transdermal enhancer, 5 g of propylene glycol, and 100 g of ethyl acetate were added. The jar was sealed and oscillated on the oscillator for 15 hrs, and then stood until bubbles disappeared. The resulting mixture was coated to a backing layer comprising poly urethane to yield a viscous polymer layer which was further dried at 40°C for 4 min, at 60°C for 2 min, at 90°C for 2 min, and then cooled. A non-adhesive paper was coated on the other surface of the viscous polymer layer. The product was cut into patches with an area of 2 cm² (1 cm × 2 cm) to yield an antiviral transdermal absorbing patch VIII with 6 mg of entecavir per square centimeter. The antiviral transdermal absorbing patch VIII comprised 82 wt.% polyacrylate pressure sensitive adhesive, 1 wt.% entecavir, 4.5 wt.% laurocapram, 6.5 wt.% eucalyptus oil, and 6 wt.% propylene glycol. The permeation rate of the patch measured using upgraded Franz diffusion cell was 15 µg/cm²/h, and the release time of the active ingredients exceeded 72 hrs. Thus, the patch was suggested for use of up to 3 days.

The following is the ninth example.

To a jar, 200 g of polyacrylate pressure sensitive adhesive (EUDRAGIT E100, from Germany, pre-dissolved with ethyl acetate, in which solid EUDRAGIT E100 accounts for 40 wt.%) as a viscous polymer, 1 g of entecavir as an antiviral agent, a mixture of 8 g of laurocapram and 8.5 g of eucalyptus oil as a transdermal enhancer, and 100 g of ethyl acetate as a solvent were added. The jar was sealed and oscillated on the oscillator for 15 hrs, and then stood until bubbles disappeared. The resulting mixture was coated to a backing layer comprising polythene-aluminum-polyethylene to yield a viscous polymer layer which was further dried at 40°C for 4 min, at 60°C for 2 min, at 90°C for 2 min, and then cooled. A non-adhesive paper was coated on the other surface of the viscous polymer layer. The product was cut into patches with an area of 2 cm² (1 cm × 2 cm) to yield an antiviral transdermal absorbing patch IX with 7 mg of entecavir per square centimeter. The antiviral transdermal absorbing patch IX comprised 82 wt.% polyacrylate pressure sensitive adhesive, 1.5 wt.% entecavir, 8 wt.% laurocapram, and 8.5 wt.% eucalyptus oil. The permeation rate of the patch measured using upgraded Franz diffusion cell was 12 µg/cm²/h, and the release time of the active ingredients exceeded 96 hrs. Thus, the patch was suggested for use of up to 4 days.

The following is the tenth example.

To a jar, 150 g of polyacrylate pressure sensitive adhesive (EUDRAGIT E100, from Germany, pre-dissolved with ethyl acetate, in which solid EUDRAGIT E100 accounts for 40 wt.%) as a viscous polymer, 0.5 g of entecavir as an antiviral agent, a mixture of 10 g of laurocapram and 10 g of eucalyptus oil as a transdermal enhancer, 9.5 g of propylene glycol, and 100 g of ethyl acetate were added. The jar was sealed and oscillated on the oscillator for 15 hrs, and then stood until bubbles disappeared. The resulting mixture was coated to a backing layer comprising low density polythene to yield a viscous polymer layer which was further dried at 40°C for 4 min, at 60°C for 2 min, at 90°C for 2 min, and then cooled. A layer of polyisobutylene was coated on the other surface of the viscous polymer layer, dried and cooled following the process mentioned above, and then a non-adhesive paper was coated. The product was cut into patches with an area of 1 cm² (1 cm × 1 cm) to yield an antiviral transdermal absorbing patch X with 0.8 mg of entecavir per square centimeter. The antiviral transdermal absorbing patch X comprised 66.4 wt.% polyacrylate pressure sensitive adhesive, 0.6 wt.% entecavir, 11 wt.% laurocapram, 11 wt.% eucalyptus oil, and 11 wt.% propylene glycol. The permeation rate of the patch measured using upgraded Franz diffusion cell was 22 µg/cm²/h, and the release time of the active ingredients exceeded 72 hrs. Thus, the patch was suggested for use of up to 3 days.

Now some test examples are illustrated.

The follow is the first test example, that is, Irritation test of entecavir transdermal patch.

Drug to be tested: entecavir.

Patch to be tested: a patch prepared by Example 7.

Patch for the control: an excipient prepared by Example 7 but no entecavir and transdermal enhancer added.

Method: 40 healthy guinea pigs (180 g ± 20 g), half male and half female, were collected and carried out with left/right self comparison experiments. At 24 hrs prior to the experiments, the hair of drug administration area (on the back) of guinea pigs was removed, with an area of 3 cm × 3 cm at both sides. The patch to be tested was pasted on one side and the excipient pasted on the other side. The patch and the excipient were pasted 8 hrs per day in 3 consecutive weeks. After pasting operation has finished, the patch and the excipient were removed, the drug administration area was cleaned with warm water or a non-irritating solvent. After one hour of patch removal as well as prior to next pasting, the guinea pigs were examined whether there were erythema, edema, pigmentation, blood spots, rough skin, or thin skin in the drug administration area. After 30-60 min, 24 hrs, 48 hrs, and 72 hrs of the final patch removal, the guinea pigs were examined with naked eyes whether there were erythema and edema in the drug administration area.

Results and conclusions: During the experiments, the appearance, behavior, feeding, and excretion of the guinea pigs were as normal as before, no abnormal pruritus occurred. The body weight of all guinea pigs increased normally, and no erythema, edema, pigmentation, blood spots, rough skin, or thin skin observed in the drug administration area. During the administration to the observation, the general condition of all guinea pig are sound, no guinea pig died. Thus, the patch had no obvious irritation.

The follow is the second test example, that is, Irritation test of lamivudine transdermal patch.

Drug to be tested: lamivudine.

Patch to be tested: a patch prepared by Example 3.

Patch for the control: an excipient prepared by Example 3 but no lamivudine and transdermal enhancer added.

Method: 30 healthy rabbits, half male and half female, were collected and carried out with left/right self comparison experiments. At 24 hrs prior to the experiments, the hair of drug administration area (on the back) of the rabbits was removed, with an area of 4 cm × 4 cm at both sides. The patch to be tested was pasted on one side and the excipient pasted on the other side. The patch and the excipient were pasted for 8 hrs per day in 3 consecutive weeks. After pasting operation has finished, the patch and the excipient were removed, the drug administration area was cleaned with warm water or a non-irritating solvent. After one hour of patch removal as well as prior to next pasting, the rabbits were examined whether there were erythema, edema, pigmentation, blood spots, rough skin, or thin skin in the drug administration area. After 30-60 min, 24 hrs, 48 hrs, and 72 hrs of the final patch removal, the rabbits were examined with naked eyes whether there were erythema and edema in the drug administration area.

Results and conclusions: During the experiments, the appearance, behavior, feeding, and excretion of the rabbits were as normal as before, no abnormal pruritus occurred. The body weight of all rabbits increased normally, and no erythema, edema, pigmentation, blood spots, rough skin, or thin skin observed in the drug administration area. During the administration to the observation, the general condition of all guinea pig are sound, no rabbit died. Thus, the patch had no obvious irritation.

The follow is the third test example, that is, Irritation test of stavudine transdermal patch.

Drug to be tested: stavudine.

Patch to be tested: a patch prepared by Example **4.**

Patch for the control: an excipient prepared by Example **4** but no stavudine and transdermal enhancer added.

Method: 30 healthy rabbits, half male and half female, were collected and carried out with left/right self comparison experiments. At 24 hrs prior to the experiments, the hair of drug administration area (on the back) of the rabbits was removed, with an area of 4 cm × 4 cm at both sides. The patch to be tested was pasted on one side and the excipient pasted on the other side. The patch and the excipient were pasted for 8 hrs per day in 3 consecutive weeks. After pasting operation has finished, the patch and the excipient were removed, the drug administration area was cleaned with warm water or a non-irritating solvent. After one hour of patch removal as well as prior to next pasting, the rabbits were examined whether there were erythema, edema, pigmentation, blood spots, rough skin, or thin skin in the drug administration area. After 30-60 min, 24 hrs, 48 hrs, and 72 hrs of the final patch removal, the rabbits were examined with naked eyes whether there were erythema and edema in the drug administration area.

Results and conclusions: During the experiments, the appearance, behavior, feeding, and excretion of the rabbits were as normal as before, no abnormal pruritus occurred. The body weight of all rabbits increased, and no erythema, edema, pigmentation, blood spots, rough skin, or thin skin observed in the drug administration area. During the administration to the observation, the general condition of all guinea pig are sound, no rabbit died. Thus, the patch had no obvious irritation.

The follow is the fourth test example, that is, Allergy test of entecavir transdermal patch.

Drug to be tested: entecavir.

Patch to be tested: a first patch whose entecavir concentration was four times that of a patch prepared by Example 7 as a sensitizer; a second patch whose entecavir concentration was eight times that of a patch prepared by Example 7 as an activator.

Negative control group: an excipient prepared by Example 7 but no entecavir and transdermal enhancer added.

Positive control group: 1% mercapto thiazole as a sensitizer and 2% mercapto thiazole as an activator.

Method: 30 healthy adult guinea pigs, either male or female, were divided into an experimental group comprising 20 adult guinea pigs, a positive control group comprising 5adult guinea pigs, and a negative control group comprising 5 adult guinea pigs. At 24 hrs prior to the experiments, the hair of drug administration area of the guinea pigs was removed, with an area of 4 cm × 4 cm at both sides. Buehler test (BT) was carried out as follows. The guinea pigs were administered with a sensitizer for 10-14 days to induce immunoreactions, and then an activator was administered. Observe whether there were allergic reactions. The skin response and response degree thereof at the induction period and attack period were compared, and the results were compared with those of the excipient group. During the Buehler test (BT), entecavir transdermal patches were pasted at the 0, 6^{th}, and 13^{th} days partially for induction, and at the 27^{th} day pasted on the non-administration area of abdomen for 6 hrs for activation. At one hour and 24 hrs after sensitization and 24 and 48 hrs after activation, observe whether there were erythema, edema, and other abnormal reactions, and evaluate the erythema and edema.

Results and conclusions: The results and conclusions were listed as Table **1**.

**Table 1**

| Skin allergy rate (%) | | | | | |
|---|---|---|---|---|---|
| Groups | 0 h | 24 h | 48 h | 72 h | Evaluation on sensitization |
| Mercapto thiazole | 60 | 80 | 90 | 80 | Allergenic |
| Negative control | 0 | 0 | 0 | 0 | Nonallergenic |
| Entecavir | 0 | 0 | 0 | 0 | Nonallergenic |

The results showed that, the patch of the invention had no obvious allergenicity.

While particular embodiments of the invention have been shown and described, it will be obvious to those skilled in the art that changes and modifications may be made without departing from the invention in its broader aspects, and therefore, the aim in the appended claims is to cover all such changes and modifications as fall within the true spirit and scope of the invention.

## Claims

1. An antiviral transdermal absorbing patch comprising a backing layer, a viscous polymer layer, and a protection film layer, wherein the viscous polymer layer comprises an antiviral agent, a viscous polymer, and a transdermal enhancer.

2. The antiviral transdermal absorbing patch according to claim 1, wherein the antiviral agent is a nucleoside antiviral drug with a daily administration rate of less than 100 mg/day.

3. The antiviral transdermal absorbing patch according to claim 2, wherein the antiviral nucleoside drug with a daily administration rate of less than 100 mg/day is selected from the group consisting of entecavir, adefovir dipivoxil, lamivudine, stavudine, or a mixture thereof.

4. The antiviral transdermal absorbing patch according to any one of claim 1 to claim 3, wherein the content of the antiviral agent per square centimeter of the antiviral transdermal absorbing patch is between 0.1 and 50 mg.

5. The antiviral transdermal absorbing patch according to any one of claim 1 to claim 3, wherein the antiviral agent accounts for between 0.1 and 15.0 wt.% of the viscous polymer layer.

6. The antiviral transdermal absorbing patch according to any one of claim 1 to claim 3, wherein the viscous polymer is selected from the group consisting of a polyacrylate pressure sensitive adhesive, polyisobutylene, polyisoprene, and a silicone copolymer.

7. The antiviral transdermal absorbing patch according to claim 6, wherein the polyacrylate pressure sensitive adhesive is selected from the group consisting of polyacrylic resin II, polyacrylic resin III, polyacrylic resin IV, ammonio methacrylate copolymer I, and ammonio methacrylate copolymer II.

8. The antiviral transdermal absorbing patch according to claim 6 or claim 7, wherein the viscous polymer accounts for between 50.0 and 96.0 wt.% of the viscous polymer layer.

9. The antiviral transdermal absorbing patch according to any one of claim 1 to claim 3, wherein the transdermal enhancer is selected from the group consisting of laurocapram, essential oils, dimethyl sulfoxide, thymol, eucalyptus oil, a traditional chinese medicine comprising terpenes or phenols, or a mixture thereof.

10. The antiviral transdermal absorbing patch according to claim 9, wherein the transdermal enhancer is laurocapram.

11. The antiviral transdermal absorbing patch according to claim 9, wherein the transdermal enhancer is a mixture of laurocapram and essential oil with a weight ratio of 1: 0.5-2.

12. The antiviral transdermal absorbing patch according to claim 9, wherein the transdermal enhancer is a mixture of laurocapram and dimethyl sulfoxide with a weight ratio of 1: 0.5-2.

13. The antiviral transdermal absorbing patch according to claim 9, wherein the transdermal enhancer accounts for between 2.0 and 12.0 wt.% of the viscous polymer layer.

14. The antiviral transdermal absorbing patch according to any one of claim 1 to claim3, wherein the viscous polymer layer further comprises an organic solvent.

15. The antiviral transdermal absorbing patch according to any one of claim 1 to claim 3, wherein an adhesive layer is disposed between the viscous polymer layer and the protection film layer.

16. The antiviral transdermal absorbing patch according to claim 15, wherein a raw material of the adhesive layer is selected from a polyacrylate pressure sensitive adhesive, polyisobutylene, or a silicone copolymer.

17. The antiviral transdermal absorbing patch according to any one of claim 1 to claim 3, wherein the backing layer is selected from the group consisting of high-density polyethylene, low density polyethylene, polypropylene, polyvinyl chloride, ethylene -vinyl acetate copolymer, polyester, polyvinylpyrrolidone, polyvinyl alcohol, poly urethane, aluminum foil, or a mixture thereof.

18. The antiviral transdermal absorbing patch according to claim 17, wherein the backing layer is a composite film formed by reaction of an aluminum foil with high-density polyethylene, low density polyethylene, polypropylene, polyvinyl chloride, ethylene-vinyl acetate copolymer, polyester, polyvinylpyrrolidone, polyvinyl alcohol, poly urethane, or a mixture thereof.

19. The antiviral transdermal absorbing patch according to claim 17, wherein the backing layer is a three-layered composite film with an aluminum foil in the middle and low density polyethylene, poly urethane, or a mixture thereof at both sides.

20. A method for producing the antiviral transdermal absorbing patch according to any one of claim 1 to claim 19, comprising the steps of: a) uniformly mixing the antiviral agent, the viscous polymer, the transdermal enhancer and a solvent to yield a mixture; b) coating the mixture to the backing layer to yield the viscous polymer layer; c) drying the viscous polymer layer and then cooling the viscous polymer layer; and d) after cooling, coating the protection film layer to the other surface of the viscous polymer layer.

21. An application of the antiviral transdermal absorbing patch according to any one of claim 1 to claim 19 in the manufacture of antiviral drug.
